# FASCICULE DE BREVET EUROPEEN

(11) **EP 1 872 731 B1**
(45) Date de publication et mention de la délivrance du brevet: **21.04.2010**
(21) Numéro de dépôt: 07012479.7
(22) Date de dépôt: 26.06.2007
(51) Int. Cl.: A61B 17/70

(54) **Implant destiné à la stabilisation de la région lombo-sacrée**
Implantat zur Stabilisierung der Lumbosakralregion
Implant intended for stabilising the lumbo-sacral region

(30) Priorité: 26.06.2006 FR 0605694
(43) Date de publication de la demande: 02.01.2008
(73) Titulaire: ARCA MEDICA GmbH, 7935 Neuenburg am Rhein (DE)
(72) Inventeur: Frydrich, Andreas, 61348 Bad Homburg (DE); Laurent, Benoît, 68100 Mulhouse (FR)
(74) Mandataire: Maillet, Alain

(56) Documents cités:
- WO-A-03/077806
- WO-A-2005/044152
- WO-A2-02/49539
- FR-A- 2 681 525
- FR-A1- 2 722 980
- US-A1- 2003 191 470
- US-B1- 6 364 883

## Description

La présente invention concerne un implant pour la stabilisation de la région lombo-sacrée, c'est-à-dire destiné à être placé entre la dernière vertèbre lombaire, la vertèbre L5, et la première vertèbre sacrée, la vertèbre S1.

D'une manière générale, les implants intervertébraux sont utilisés pour remédier à des problèmes de dos et pour soulager certaines douleurs. Les implants intervertébraux trouvent en particulier application dans le cadre de problèmes de déstabilisation vertébrale. La déstabilisation vertébrale peut provoquer un mouvement anormal de la colonne vertébrale et engendrer une douleur ressentie par le patient. Dans le cas d'une hernie discale, par exemple, la dégradation d'un disque intervertébral peut provoquer un jeu anormal des vertèbres attenantes à ce disque. Une solution proposée pour remédier à ces problèmes d'instabilité des vertèbres consiste alors à implanter un implant pour stabiliser le rachis.

Un certain nombre d'implants intervertébraux ont été développés depuis de nombreuses années. Les implants sont généralement constitués d'éléments rigides pourvus de moyens d'ancrage osseux.

Certains implants sont prévus pour être fixés en partie supérieure sur l'apophyse épineuse d'une vertèbre et en partie inférieure sur l'apophyse épineuse d'une vertèbre inférieure. Il existe, par exemple, un implant constituant une cale que l'on dispose entre les apophyses épineuses de deux vertèbres consécutives. Une telle cale est formée par deux oreilles faisant saillie de la portion inter-épineuse de la cale et délimitant chacune un évidement dans lequel vient se loger l'apophyse épineuse de chaque vertèbre. Cette cale est maintenue en position par un système de ligamcnts entourant les apophyses épineuses des vertèbres. L'inconvénient majeur de ce dispositif est qu'il est difficilement implantable dans la région lombo-sacrée du fait de l'anatomie soudée des vertèbres sacrées chez lesquelles l'apophyse épineuse ne forme plus qu'une légère bosse.

Le document FR-A1-2 722 980 décrit les caractéristiques du préambule de la revendication 1.

On trouve également des implants sous forme de tiges métalliques ou de plaquettes rigides que l'on fixe, par exemple, de part et d'autre des apophyses épineuses le long de plusieurs vertèbres grâce à des vis. La mise en place de ces implants est relativement complexe et la fixation des tiges le long des vertèbres a pour conséquence de limiter, voire même empêcher, tout mouvement de la portion vertébrale concernée.

D'autres implants se présentent sous la forme de différentes pièces, assemblées les unes aux autres par le chirurgien au fur et à mesure de leur implantation, et prévues pour être ancrées, par l'intermédiaire de moyens d'accrochage, sur les apophyses ou les lames des vertèbres. Ces implants présentent l'inconvénient d'être difficiles et longs à mettre en place du fait du grand nombre de pièces devant être assemblées.

Le but de la présente invention est de proposer un nouvel implant qui puisse être implanté dans la région lombo-sacrée et qui soit composé d'un nombre réduit de pièces de manière à faciliter sa mise en place.

A cet effet, un implant destiné à la stabilisation de la région lombo-sacrée selon l'invention est caractérisé en ce qu'il comprend un corps central de section en U, deux ailerons parallèles entre eux faisant saillie de la face extérieure d'une branche dudit corps central et formant ensemble un moyen d'ancrage à l'apophyse épineuse de la dernière vertèbre lombaire, ledit moyen d'ancrage présentant une section en U, et deux pinces de section en U disposées de part et d'autre de l'autre branche dudit corps central et chacune destinée à être respectivement ancrée sur les rebords supérieurs des lames de la première vertèbre sacrée.

L'implant selon l'invention se composc ainsi d'un nombre réduit de pièces et chacune des pièces est prévue pour être facilement ancrée sur la partie osseuse de la région lombo sacrée lui correspondant.

L'implant selon l'invention est également parfaitement adapté à être mis en place entre la vertèbre L5 et la vertèbre S1 de la région lombo sacrée. En effet, le moyen d'ancrage de section en U formé par les deux ailerons est prévu pour être ancré sur l'apophyse épineuse de la vertèbre L5 et les deux pinces de section en U sont prévues pour être ancrées chacune sur le rebord supérieur d'une lame de la vertèbre SI.

Selon une caractéristique de l'invention, l'implant est monobloc.

L'implant se présente ainsi sous la forme d'une seule pièce ce qui en facilite la fabrication et la manipulation lors de la mise en place.

Selon une caractéristique de l'invention, l'implant est réalisé en titane.

Selon un mode de réalisation de l'invention, le coude du corps central est réalisé en matière élastique.

Avantageusement, le coude du corps central est en caoutchouc.

Selon une autre caractéristique de l'invention, les longueurs des branches dudit corps central sont prévues pour que lorsque ledit moyen d'ancrage formé par lesdits ailerons est ancré à l'apophyse épineuse de la dernière vertèbre lombaire, lesdits crochets peuvent être ancrés sur les rebords supérieurs des lames de la première vertèbre sacrée, respectivement.

Selon un mode de réalisation de l'invention, ledit corps central présente au moins un moyen de fixation à l'apophyse épineuse.

Selon une caractéristique de l'invention, ledit moyen de fixation est constitué par au moins un cran formé dans la face extérieure de ladite branche d'où font saillie lesdits deux ailerons et en arrière desdits ailerons et prévu pour être placé contre la face inférieure de l'apophyse épineuse de la dernière vertèbre lombaire.

Selon une caractéristique de l'invention, chacun desdits ailerons comporte au moins un moyen de fixation à l'apophyse épineuse de la dernière vertèbre lombaire.

Selon une caractéristique de l'invention, ledit moyen de fixation est constitué par au moins un cran perpendiculaire au plan dudit aileron, formé dans sa face intérieure, et prévu pour être placé contre la face latérale de l'apophyse épineuse de la dernière vertèbre lombaire.

De façon avantageuse, chacun desdits ailerons comporte au moins un orifice propre à loger un moyen de forme complémentaire.

Selon une autre caractéristique de l'invention, chacune desdites pinces forme avec ledit corps central un angle aigu.

Selon une autre caractéristique de l'invention, les parois de chacune desdites pinces forment entre elles un angle aigu.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
La Fig. 1 représente une vue de profil d'un implant selon l'invention.
La Fig. 2 représente une autre vue de profil d'un implant selon l'invention.
La Fig. 3 représente un implant selon l'invention implanté dans la région lombo sacrée en vue de face.
La Fig. 4 représente un implant selon l'invention implanté dans la région lombo sacrée en vue de profil.

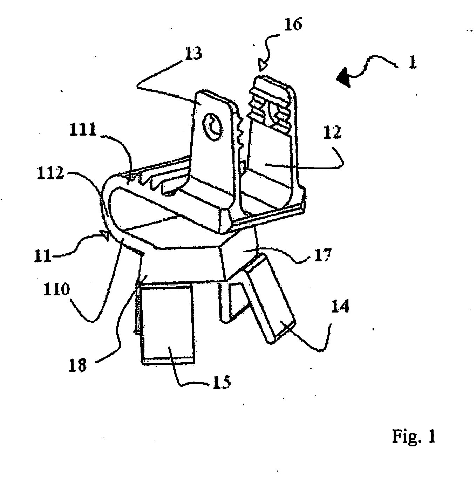

A la Fig. 1 est représenté un implant 1 destiné à la stabilisation de la région lombo-sacrée selon l'invention. L'implant 1 est composé d'un corps central 11 comprenant deux branches 111 et 110 reliées entre elles par un coude 112. Le corps central 11 forme ainsi une section en U.

A l'extrémité de la face extérieure d'une des branches, la branche 111 du corps central 11, font saillie deux ailerons 12 et 13 parallèles entre eux. Les ailerons 12 et 13 sont perpendiculaires au plan du corps central 11 et forment ensemble un moyen d'ancrage 16 prévu pour recevoir l'apophyse épineuse de la dernière vertèbre lombaire L5.

L'autre branche du corps central 11, la branche 110, présente de part et d'autre de son extrémité deux pinces 14 et 15. La pince 14 est reliée à la branche 110 par l'intermédiaire d'un élément de liaison 17 et la pince 15 est reliée à la branche 110 par l'intermédiaire d'un élément de liaison 18. Les éléments de liaison 17 et 18 permettent d'orienter les pinces 14 et 15 selon un angle aigu formé avec la branche 110. Les pinces 14 et 15 sont prévues pour s'ancrer respectivement sur les rebords supérieurs des deux lames de la première vertèbre sacrée SI.

L'implant 1 présente la caractéristique d'être monobloc. En effet, le corps central 11, la pince 14, la pince 15 et le moyen d'ancrage 16 forment ensemble une pièce unique.

L'implant 1 est généralement réalisé entièrement en titane mais le coude 112 du corps central 11 peut être composé de matière élastique, tel que du caoutchouc. Le titane présente la particularité d'être biocompatible, c'est-à-dire qu'il ne provoque normalement pas d'infection à l'intérieur du corps humain. Le titane présente également la caractéristique de ne pas dégrader les images obtenues par des procédés d'imagerie tels que la radiographie ou le scanner.

La présence de matière élastique dans le coude 112 permet d'absorber les contraintes mécaniques exercées sur les parties osseuses des vertèbres entre lesquelles l'implant est disposé. La matière élastique permet également de donner à l'implant la souplesse nécessaire pour permettre la flexion et l'extension de la région lombo-sacrée.

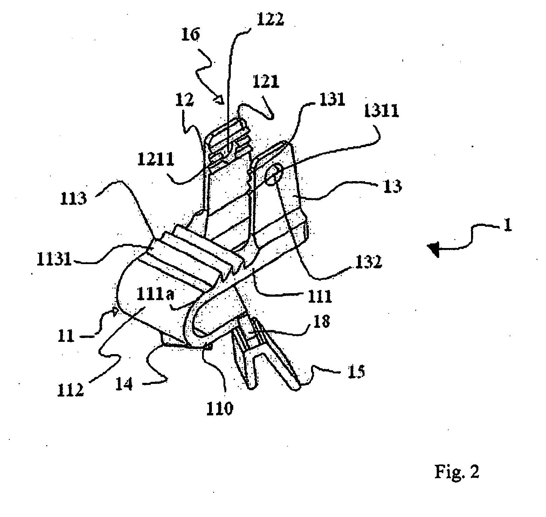

La branche 111 du corps central 11 de l'implant 1, représenté à la Fig. 2, présente sur sa face externe 111a un moyen de fixation 113 destiné à fixer le corps central 11 à l'apophyse épineuse de la vertèbre lombaire L5. Le moyen de fixation 113 est, de façon avantageuse, constitué par au moins un cran 1131. Le cran 1131 est formé dans la face externe 111a de la branche 111. Les crans 113 sont inclinés dans la direction du moyen d'ancrage 16.

Les ailerons 12 et 13 qui forment le moyen d'ancrage 16 comportent respectivement des moyens de fixation 121 et 131 destinés à fixer le moyen d'ancrage 16 sur l'apophyse épineuse de la dernière vertèbre lombaire. Les moyens de fixation 121 et 131 sont constitués par au moins un cran, tel que les crans 1211 et 1311, qui sont respectivement formés dans les faces intérieures des ailerons 12 et 13 et qui sont, de plus, respectivement perpendiculaires au plan des ailerons 12 et 13.

Chacun des ailerons 12 et 13 comporte respectivement au moins un orifice 122 et 132. Les orifices 122 et 132 sont prévus pour loger des moyens présentant une forme complémentaire, telle qu'une vis ou une tige de fixation à l'os.

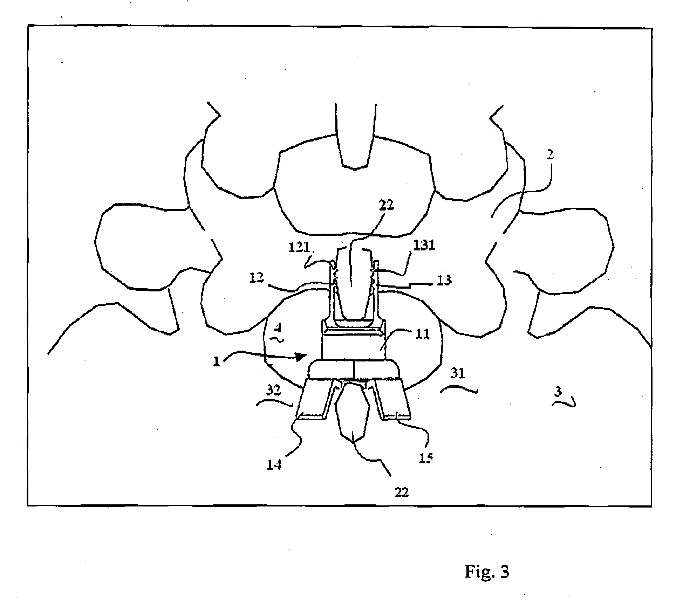

La Fig. 3 représente l'implant 1 selon l'invention une fois placé dans la région lombo-sacrée d'un patient. Le corps central 11 de l'implant 1 est disposé dans l'espace 4 créé entre la vertèbre lombaire L5, référencée 2 sur la figure, et la vertèbre sacrée S1, référencée 3 sur la figure. L'implant 1 est disposé de façon à ce que le coude 112 du corps central 11 soit situé à l'intérieur de l'espace 4.

Le moyen d'ancrage 16, formé par les ailerons 12 et 13, présente une section de forme en U qui permet de recevoir l'apophyse épineuse 22 de la vertèbre lombaire 2.

Les pinces 14 et 15 sont ancrées sur les rebords supérieurs des lames 32 et 31 de la vertèbre sacrée S1, respectivement, grâce à l'angle aigu que chacune forme avec la branche 110 du corps central 1. Ces angles aigus s'avèrent nécessaires du fait de l'anatomie de la vertèbre sacrée 3.

Les crans qui constituent les moyens de fixation 121 et 131 des ailerons 12 et 13, respectivement, sont appliqués contre les faces latérales de l'apophyse épineuse 22 et permettent d'empêcher le moyen d'ancrage 16 de se déplacer dans une direction perpendiculaire au plan de l'apophyse épineuse 22.

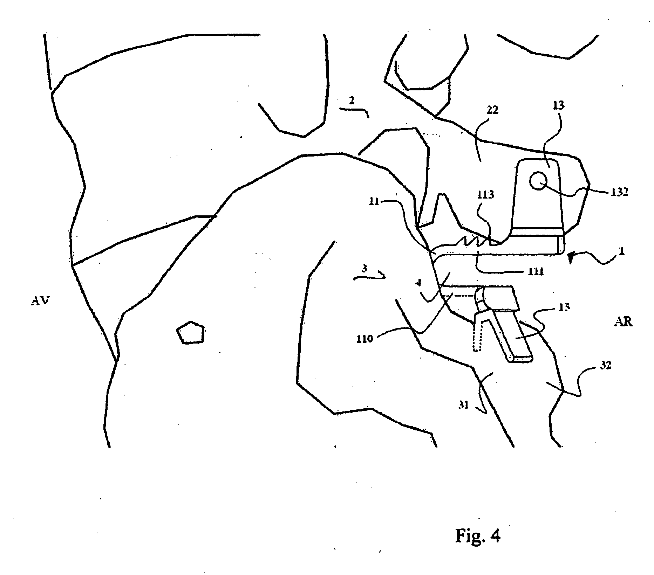

La Fig. 4 permet de distinguer de façon précise le sens d'implantation de l'implant 1 selon l'invention. L'implant 1 est disposé de façon à ce que le coude 112 soit dirigé vers l'avant AV des vertèbres, à l'intérieur de l'espace 4 formé entre la vertèbre lombaire 2 et la vertèbre sacrée 3.

Les moyens de fixation 113 constitués par des crans et formés dans la face extérieure 111a de la branche 111 du corps central 11 sont disposés contre la face inférieure de l'apophyse épineuse de la vertèbre lombaire 2. Les moyens de fixation 113 permettent d'empêcher l'implant 1 de se déplacer dans une direction parallèle au plan de l'apophyse épineuse 22 de la vertèbre lombaire 2, vers l'arrière AR de la vertèbre 2.

Les longueurs des branches 111 et 110 du corps central 11 sont prévues pour que lorsque le moyen d'ancrage 16 est ancré à l'apophyse épineuse 22 de la vertèbre lombaire 2, les pinces 14 et 15 puissent être respectivement ancrées sur les rebords supérieurs respectifs des lames 31 et 32 de la vertèbre sacrée 3. Ainsi, la longueur de la branche 111 est supérieure à la longueur de la branche 110.

L'orifice 132 de l'aileron 13, ainsi que l'orifice 122 de l'aileron 12 (non visible sur la figure), sont prévus pour permettre la fixation des ailerons 12 et 13 sur l'apophyse épineuse 22. La forme des orifices 122 et 132 permet le passage d'un élément de fixation tel qu'une vis prévue pour être vissée dans l'os.

L'implant 1 étant monobloc, sa disposition dans la région lombo-sacrée est facilitée et simplifiée.

## Revendications

1. Implant (1) destiné à la stabilisation de la région lombo-sacrée, comprenant un corps central (11) de section en U, deux ailerons (12, 13) parallèles entre eux faisant saillie de la face extérieure d'une branche (111) dudit corps central et formant ensemble un moyen d'ancrage (16) à l'apophyse épineuse (22) de la dernière vertèbre lombaire (2), ledit moyen d'ancrage 16 présentant une section en U, **caractérisé en ce qu'**il comporte deux pinces (14, 15) de section en U disposées de part et d'autre de l'autre branche (110) dudit corps central (11) et chacune destinée à être ancrée respectivement sur les rebords supérieurs des lames (31, 32) de la première vertèbre sacrée (3).

2. Implant (1) selon la revendication 1, **caractérisé en ce qu'**il est monobloc.

3. Implant (1) selon l'une des revendications précédentes, **caractérisé en ce** les longueurs des branches (111, 110) dudit corps central (11) sont prévues pour que lorsque ledit moyen d'ancrage (16) formé par lesdits ailerons (12, 13) est ancré à l'apophyse épineuse (22) de la dernière vertèbre lombaire (2), lesdites pinces (14, 15) peuvent être respectivement ancrés sur les rebords supérieurs des lames (31) et (32) de la première vertèbre sacrée (3),

4. Implant (1) selon l'une des revendications précédentes, **caractérisé en ce que** ledit corps central (11) présente au moins un moyen de fixation (113) à l'apophyse épineuse (22).

5. Implant (1) selon la revendication 4, **caractérisé en ce que** ledit moyen de fixation (113) est constitué par au moins un cran (1131) qui est formé sur la face extérieure (111a) de ladite branche (111) d'où font saillie lesdits ailerons (12; 13) et en arrière desdits ailerons (12, 13) et qui est prévu pour être placé contre la face inférieure de l'apophyse épineuse (22) de la dernière vertèbre lombaire (2).

6. Implant (1) selon l'une des revendications précédentes, **caractérisé en ce que** chacun desdits ailerons (12, 13) comporte au moins un moyen de fixation (121, 131) à l'apophyse épineuse (22) de la dernière vertèbre lombaire (2).

7. Implant (1) selon la revendication 6, **caractérisé en ce que** ledit moyen de fixation (131, 121) est constitué par au moins un cran (1311) perpendiculaire au plan dudit aileron (12, 13) et formé dans sa face intérieure et prévu pour être placé contre la face latérale de l'apophyse épineuse (22) de la dernière vertèbre lombaire (2).

8. Implant (1) selon l'une des revendications précédentes, **caractérisé en ce que** chacun desdits ailerons (12, 13) comporte au moins un orifice (122, 132) propre à loger un moyen de forme complémentaire.

9. Implant (1) selon l'une des revendications précédentes, **caractérisé en ce que** chacune desdites pinces (14, 15) forme avec ledit corps central (11) un angle aigu.

10. Implant (1) selon l'une des revendications précédentes, **caractérisé en ce que** les parois de chacune desdites pinces (14,15) forment entre elles un angle aigu.

11. Implant (1) selon l'une des revendications précédentes, **caractérisé en ce qu'**il est réalisé en titane.

12. Implant (1) selon l'une des revendications précédentes, **caractérisé en ce que** le coude (112) dudit corps central (11) est réalisé en matière élastique.

13. Implant (1) selon la revendication 12, **caractérisé en ce que** ledit coude (112) est en caoutchouc.

## Claims

1. Implant (1) intended for stabilising the lumbo-sacral region, comprising a central body (11) with a U section, two fins (12, 13) parallel to one another projecting from the external surface of a branch (111) of the said central body and together forming a means of anchorage (16) to the spinous process of vertebra (22) of the last lumbar vertebra (2), the said means of anchorage 16 presenting a U section, **characterised in that** it includes two clamps (14, 15) with a U section arranged on either side of the other branch (110) of the said central body (11) and each intended to be anchored respectively to the upper edges of the blades (31, 32) of the first sacral vertebra (3).

2. Implant (1) according to claim 1, **characterised in that** it is a one-piece unit.

3. Implant (1) according to one of the preceding claims, **characterised in that** the lengths of the branches (111, 110) of the said central body (11) are such that, when the said means of anchorage (16) formed by the said fins (12, 13) is anchored to the spinous process of vertebra (22) of the last lumbar vertebra (2), the said clamps (14, 15) can be respectively anchored to the upper edges of the blades (31) and (32) of the first sacral vertebra (3).

4. I mplant (1) according to one of the preceding claims, **characterised in that** the said central body (11) presents at least one means of fixing (113) to the spinous process of vertebra (22).

5. I mplant (1) according to claim 4, **characterised in that** the said means of fixing (113) is made up of at least one notch (1131) formed on the external surface (111a) of the said branch (111) from which the said fins project (12, 13) and behind the said fins (12, 13) and which is intended to be placed against the lower surface of the spinous process of vertebra (22) of the last lumbar vertebra (2).

6. I mplant (1) according to one of the preceding claims, **characterised in that** each of the said fins (12, 13) includes at least one means of fixing (121, 131) to the spinous process of vertebra (22) of the last lumbar vertebra (2),

7. I mplant (1) according to claim 6, **characterised in that** the said means of fixing (131, 121) is made up of at least one notch (1311) perpendicular to the plane of the said fin (12, 13) and formed in its internal surface and intended to be placed against the lateral surface of the spinous process of vertebra (22) of the last lumbar vertebra (2).

8. I mplant (1) according to one of the preceding claims, **characterised in that** each of the said fins (12, 13) includes at least one orifice (122, 132) able to house a means of a complementary shape.

9. I mplant (1) according to one of the preceding claims, **characterised in that** each of the said clamps (14, 15) forms an acute angle with the said central body (11).

10. Implant (1) according to one of the preceding claims, **characterised in that** the walls of each of the said clamps (14, 15) form an acute angle between then.

11. Implant (1) according to one of the preceding claims, **characterised in that** it is made from titanium.

12. Implant (1) according to one of the preceding claims, **characterised in that** the elbow (112) of the said central body (11) is made from an elastic material.

13. Implant (1) according to claim 12, **characterised in that** the said elbow (112) is made from rubber.

## Patentansprüche

1. Implantat (1) zur Stabilisierung der Lumbosakralregion, umfassend einen Mittelkörper (11) mit U-förmigem Querschnitt, zwei zueinander parallele Flügel (12, 13), die von der Außenfläche eines Schenkels (111) des Mittelkörpers vorstehen und gemeinsam ein Mittel zur Verankerung (16) am Dornfortsatz (22) des letzten Lendenwirbels (2) bilden, wobei das Mittel zur Verankerung (16) einen U-förmigen Querschnitt aufweist, **dadurch gekennzeichnet, dass** es zwei Klammern (14, 15) mit U-förmigem Querschnitt aufweist, die zu beiden Seiten des anderen Schenkels (110) des Mittelkörpers (11) angeordnet sind und dazu vorgesehen sind, jeweils an den oberen Rändern der Laminae (31, 32) des ersten Sakralwirbels (3) verankert zu werden.

2. Implantat (1) nach Anspruch 1, **dadurch gekennzeichnet, dass** es einteilig ist.

3. Implantat (1) nach einem der vorangegangenen Ansprüche, **dadurch gekennzeichnet, dass** die Längen der Schenkel (111, 110) des Mittelkörpers (11) derart vorgesehen sind, dass dann, wenn das von den Flügeln (12, 13) gebildete Mittel zur Verankerung (16) am Dornfortsatz (22) des letzten Lendenwirbels (2) verankert ist, die zwei Klammern (14, 15) jeweils an den oberen Rändern der Laminae (31) und (32) des ersten Sakralwirbels (3) verankert werden können.

4. Implantat (1) nach einem der vorangegangenen Ansprüche, **dadurch gekennzeichnet, dass** der Mittelkörper (11) mindestens ein Mittel zur Fixierung (113) am Dornfortsatz (22) aufweist.

5. Implantat (1) nach Anspruch 4, **dadurch gekennzeichnet, dass** das Mittel zur Fixierung (113) aus mindestens einer Einkerbung (1131) besteht, die an der Außenfläche (111a) des Schenkels (111), von der aus die Flügel (12, 13) vorstehen, hinter den Flügeln (12, 13) ausgebildet ist und dazu vorgesehen ist, an die Unterseite des Dornfortsatzes (22) des letzten Lendenwirbels (2) angelegt zu werden.

6. Implantat (1) nach einem der vorangegangenen Ansprüche, **dadurch gekennzeichnet, dass** jeder der Flügel (12, 13) mindestens ein Mittel zur Fixierung (121, 131) am Dornfortsatz (22) des letzten Lendenwirbels (2) aufweist.

7. Implantat (1) nach Anspruch 6, **dadurch gekennzeichnet, dass** das Mittel zur Fixierung (131, 121) aus mindestens einer Einkerbung (1311) besteht, die senkrecht zur Ebene des Flügels (12, 13) ist und an seiner Innenseite ausgebildet ist und dazu vorgesehen ist, an die Seitenfläche des Dornfortsatzes (22) des letzten Lendenwirbels (2) angelegt zu werden.

8. Implantat (1) nach einem der vorangegangenen Ansprüche, **dadurch gekennzeichnet, dass** jeder der Flügel (12, 13) mindestens eine Öffnung (122, 132) aufweist, die dazu geeignet ist, ein Mittel von komplementärer Form aufzunehmen.

9. Implantat (1) nach einem der vorangegangenen Ansprüche, **dadurch gekennzeichnet, dass** jede der Klammern (14, 15) mit dem Mittelkörper (11) einen spitzen Winkel bildet.

10. Implantat (1) nach einem der vorangegangenen Ansprüche, **dadurch gekennzeichnet, dass** die Wände einer jeden Klammer (14, 15) zueinander einen spitzen Winkel bilden.

11. Implantat (1) nach einem der vorangegangenen Ansprüche, **dadurch gekennzeichnet, dass** es aus Titan hergestellt ist.

12. Implantat (1) nach einem der vorangegangenen Ansprüche, **dadurch gekennzeichnet, dass** das Knie (112) des Mittelkörpers (11) aus einem elastischen Material hergestellt ist.

13. Implantat (1) nach Anspruch 12, **dadurch gekennzeichnet, dass** das Knie (112) aus Kautschuk ist.
